# EUROPEAN PATENT APPLICATION

(11) **EP 1 174 520 A2**
(43) Date of publication of application: **23.01.2002**
(21) Application number: 01114952.3
(22) Date of filing: 20.06.2001
(51) Int. Cl.: C12Q 1/68

(54) **Method for monitoring a fermentation process using an expression array**

(30) Priority: 18.07.2000 US 219030 P
(71) Applicant: Degussa AG, 40474 Düsseldorf (DE)
(72) Inventor: Farwick, Mike, Dr., 33615 Bielefeld (DE); Brehme, Jennifer, 33649 Bielefeld (DE); Hermann, Thomas, Dr., 33739 Bielefeld (DE); Bathe, Brigitte, 33154 Salzkotten (DE); Marx, Achim, Dr., 33790 Halle (DE); Möckel, Bettina, 40597 Düsseldorf (DE); Rieping, Mechthild, 33619 Düsseldorf (DE); Ermantraut, Eugen, 07745 Jena (DE); Ellinger, Thomas, 07743 Jena (DE); Huthmacher, Klaus, Dr., 63584 Gelnhausen (DE); Pfefferle, Walter, Dr., 33790 Halle (Westf.) (DE)

(57) **Abstract**

The present invention provides arrays of single- or doublestranded desoxyribonucleic acid (DNA) probes immobilized on solid supports and methods for using these probe arrays to monitor specific nucleic acid sequences contained in a biological sample especially to monitor the nucleic acid sequences involved in a fermentation process.

SEQUENCE PROTOCOL

<110> Degussa AG

<120> Method to monitore a fermentation process

<130> 000245BT

<140>

<141>

<160> 6

<170> PatentIn Ver. 2.1

<210> 1

<211> 20

<212> DNA

<213> Corynebacterium glutamicum

<220>

<223> primer: aceA1

<400> 1

ccacacctac cctgaccagt 20

<210> 2

<211> 20

<212> DNA

<213> Corynebacterium glutamicum

<220>

<223> primer: aceA2

<400> 2

ggctcgagac cattcttgac 20

<210> 3

<211> 19

<212> DNA

<213> Corynebacterium glutamicum

<220>

<223> primer: pgi-int1

<400> 3

gacctcgttt ctgtgttgg                                                    19

<210> 4

<211> 19

<212> DNA

<213> Corynebacterium glutamicum

<220>

<223> primer: pgi-int2

<400> 4

tgacttgcca tttgattcc                                                    19

<210> 5

<211> 29

<212> DNA

<213> Corynebacterium glutamicum

<220>

<223> primer: gapA1

<400> 5

tgctctagat tgaagccagt gtgagttgc                                         29

<210> 6

<211> 29

<212> DNA

<213> Corynebacterium glutamicum

<220>

<223> primer: gapA2

<400> 6

tgctctagag atgacacatc accgtgagc                                         29

## Description

The present invention provides arrays of single- or doublestranded desoxyribonucleic acid (DNA) probes immobilized on solid supports and for using those probe arrays to detect specific nucleic acid sequences contained in a target nucleic acid in a sample, especially a method to monitore a fermentation process.

### Field of the Invention

DNA probes have long been used to detect complementary nucleic acid sequences in a nucleic acid of interest (the "target" nucleic acid).

In general the DNA probe is tethered, i.e. by covalent attachment, to a solid support, and arrays of DNA probes immobilized on solid supports have been used to detect specific nucleic acid sequences in a target nucleic acid (see, e.g., PCT WO 89/10977 or 89/11548). Methods for making high density arrays of DNA probes on silica chips and for using these probe arrays are provided in U.S. Patent No. 5,837,832 and EP Patent No. 0 373 203 or EP Patent No. 0 386 229.

The so called "DNA-chips" offer great promise for a wide variety of applications. New methods and applications are required to realize this promise, and the present invention helps meet that need.

### Summary of the Invention

The genome-wide transcriptional monitoring of organisms by the DNA-chip technology opens a new level of complexity in the functional analysis of living organisms. We have used DNA-chips for the analysis of gene expression patterns in the compound producing microorganism Corynebacterium glutamicum and Escherichia coli. Based on the available sequence information DNA-fragments of the bacterium are immobilized on a solid support. Transcription profiles of the organisms are analyzed under various fermentational conditions by DNA-Microarray experiments. The obtained data are verified by Northern-Blot analysis, real time RT-PCR or two-dimensional gel electrophoresis.

Different to the classical applications of the DNA-Microarray technology, e.g. in the biomedical research, the invention provides DNA-chips to be used for the monitoring of process related target genes in the production of fermentative available compounds.

The invention provides an analysis system for the detection of microbial gene expression patterns in large-scale industrial fermentations. The information obtained from these patterns can be used for controlling of the fermentation process.

### Detailed Description of the Invention

We claim an array of DNA probes immobilized on a solid support, said array having at least 10 probes and no more than 200.000 different DNA probes 15 to 4.000 nucleotides in length occupying separate known sites in said array, said DNA probes comprising at least one probe that is exactly complementary to selected reference sequences of a compound producing microorganism.

In a preferred embodiment of the invention, said DNA probes are nucleic acids covering a genomic region of a compound producing microorganism, e.g. obtained from a genomic shotgun library.

In another preferred embodiment of the invention, said DNA probes are nucleic acids, e.g. obtained from a polymerase chain reaction, covering an whole genetic element, an internal fragment of a genetic element or the genetic element and additionally flanking regions of it.

In a preferred embodiment of the invention, said DNA-probes are single-stranded nucleic acids, e.g. obtained from an on chip synthesis or an attachment of presynthesized oligonucleotides complementary to nucleic acids of a compound producing microorganism.

In a preferred embodiment of the invention, said reference sequence is a single-stranded nucleic acid and probes complementary to the single-stranded nucleic acid or to a DNA or RNA copy (cDNA/cRNA) of the single-stranded nucleic acid of said reference are in said array. The reference sequence is a polynucleotide sequence from a compound producing strain, especially a Corynebacterium glutamicum strain or an Escherichia coli strain.

Another embodiment of the invention is a method of analyzing a polynucleotide sequence of a compound producing microorganism, by the use of an array of DNA probes immobilized on a solid support, the different DNA's occupying separate cells of the array, which method comprises labeling the polynucleotide sequence or fragments thereof, applying the polynucleotide sequence or fragments thereof under hybridization conditions to the array, and observing the location of the label on the surface associated with particular members of the set of DNA.

The DNA-chips as mentioned above can be used to study and detect different RNA sequences or fragments thereof. Therefore the polynucleotide sequence or fragments thereof or a copy of the polynucleotide sequence or fragments thereof are applied to the DNA-chip under hybridization conditions.

The sequences of the compound producing Corynebacterium or Escherichia coli can be found in different databases, e.g.:

The NCBI is the National Center for Biotechnology Information. It is the database of the National Library of Medicine, Building 38A, Room 8N805, Bethesda, MD 20894, USA. (http://www.ncbi.nlm.nih.gov/)

Swissprot and Trembl entries can be accessed from the Swiss Institute of Bioinformatics, CMU - Rue Michel-Servet 1, 1211 Genève 4, Switzerland.(http://www.expasy.ch/)

PIR is the Protein Information Resource Database of the National Biomedical Research Foundation, 3900 Reservoir Rd., NW. Washington, DC 20007, USA. (http://www-nbrf.georgetown.edu/pirwww/pirhome.shtml)

### Selected reference sequences are especially:

| **NAME** | **ACCESSION No.** | **DATABASE** |
|---|---|---|
| 16s rDNA | X84257 | NCBI |
| aceA | X75504 | NCBI |
| aceB | L27123 | NCBI |
| acn | AB025424 | NCBI |
| aroB | AF124600 | NCBI |
| aroC | AF124600 | NCBI |
| aroE | AF124518 | NCBI |
| aroK | AF124600 | NCBI |
| asd | X57226 | NCBI |
| cat | AJ132968 | NCBI |
| citE | AJ133719 | NCBI |
| clgIIR | U13922 | NCBI |
| cop1 | X66078 | NCBI |
| phage 304L int | Y18058 | NCBI |
| csp2 | X69103 | NCBI |
| cydA | AB035086 | NCBI |
| cydB | AB035086 | NCBI |
| dapA | E16749 | NCBI |
| dapB | E16752 | NCBI |
| dapD | AJ004934 | NCBI |
| dapE | X81379 | NCBI |
| dciAE | AF038651 | NCBI |
| ddh | Y00151 | NCBI |
| DNA-Sequence | E16888 | NCBI |
| DNA-Sequence | E16889 | NCBI |
| DNA-Sequence | E16890 | NCBI |
| DNA-Sequence | E16891 | NCBI |
| DNA-Sequence | E16892 | NCBI |
| DNA-Sequence | E16893 | NCBI |
| DNA-Sequence | E16894 | NCBI |
| DNA-Sequence | E16895 | NCBI |
| DNA-Sequence | E16896 | NCBI |
| dtsR1 | AB018530 | NCBI |
| efP | X99289 | NCBI |
| fda | X17313 | NCBI |
| ftsQ | E17182 | NCBI |
| ftsY | AJ010319 | NCBI |
| gap | X59403 | NCBI |
| gdhA | X59404 | NCBI |
| glnA | AF005635 | NCBI |
| glnB | AJ010319 | NCBI |
| glt | X66112 | NCBI |
| glyA | E12594 | NCBI |
| gnd | E13660 | NCBI |
| grcC | AF130462 | NCBI |
| hisE | AF086704 | NCBI |
| hom | E14598 | NCBI |
| icd | X71489 | NCBI |
| inhA | AF145898 | NCBI |
| leuA | X70959 | NCBI |
| leuB | Y09578 | NCBI |
| lmrB | AF237667 | NCBI |
| lpd | Y16642 | NCBI |
| ltsA + ORF1 | AB029550 | NCBI |
| lysA | E16358 | NCBI |
| lysC | E16745, E16746 | NCBI |
| lysE | X96471 | NCBI |
| lysG | X96471 | NCBI |
| lysI | X60312 | NCBI |
| malE | AF234535 | NCBI |
| mqo | AJ224946 | NCBI |
| murF | E14256 | NCBI |
| murI | AB020624 | NCBI |
| ndh | AJ238250 | NCBI |
| nrdE | AF112535 | NCBI |
| nrdF | AF112536 | NCBI |
| nrdH | AF112535 | NCBI |
| nrdI | AF112535 | NCBI |
| nusG | AF130462 | NCBI |
| obg | U31224 | NCBI |
| odhA | E14601 | NCBI |
| ORF4 | X95649 | NCBI |
| panB | X96580 | NCBI |
| panC | X96580 | NCBI |
| panD | AF116184 | NCBI |
| pepQ | AF124600 | NCBI |
| porA | AJ238703 | NCBI |
| proP | Y12537 | NCBI |
| ptsM | L18874 | NCBI |
| pyc | Y09548 | NCBI |
| pyk | L27126 | NCBI |
| recA | X75085 | NCBI |
| rel | Y18059 | NCBI |
| rep | AB003157 | NCBI |
| rplA | AF130462 | NCBI |
| rplK | AF130462 | NCBI |
| secA | D17428 | NCBI |
| secE | D45020, AF130462 | NCBI |
| secG | AJ007732 | NCBI |
| Seq 1 Patent EP0563527 | A78798 | NCBI |
| Seq 1 Patent WO9519442 | A45577 | NCBI |
| Seqll Patent WO9519442 | A45587 | NCBI |
| Seq 2 Patent EP0563527 | A78797 | NCBI |
| Seq 2 Patent WO9723597 | A93933 | NCBI |
| Seq 3 Patent EP0563527 | A78796 | NCBI |
| Seq 3 Patent WO9519442 | A45579 | NCBI |
| Seq 5 Patent WO9519442 | A45581 | NCBI |
| Seq 7 Patent WO9519442 | A45583 | NCBI |
| Seq 9 Patent WO9519442 | A45585 | NCBI |
| soxA | AJ007732 | NCBI |
| thrB | Y00546 | NCBI |
| tkt | AB023377 | NCBI |
| tnp | AF189147 | NCBI |
| tpi | X59403 | NCBI |
| tRNA-Thr | AF130462 | NCBI |
| tRNA-Trp | AF130462 | NCBI |
| ureA | AJ251883 | NCBI |
| - | PIR:I40724 | PIR |
| - | PIR:S18758 | PIR |
| - | PIR:S52753 | PIR |
| - | PIR:S60064 | PIR |
| argS | PIR:A49936 | PIR |
| aro | PIR:I40837 | PIR |
| aroP | PIR:S52754 | PIR |
| aspA | PIR:JC4101 | PIR |
| atpD | PIR:I40716 | PIR |
| bioA | PIR:I40336 | PIR |
| bioB | PIR:JC5084 | PIR |
| bioD | PIR:I40337 | PIR |
| cglIIR | PIR:B55225 | PIR |
| cglIR | PIR:A55225 | PIR |
| dtsR | PIR:JC4991 | PIR |
| dtxR | PIR:I40339 | PIR |
| galE | PIR:JC5168 | PIR |
| gdh | PIR:S32227 | PIR |
| hisA | PIR:JE0213 | PIR |
| hisF | PIR:JE0214 | PIR |
| ilvA | PIR:A47044 | PIR |
| ilvB | PIR:A48648 | PIR |
| ilvC | PIR:C48648 | PIR |
| pgk | PIR:B43260 | PIR |
| pheA | PIR:A26044 | PIR |
| proA | PIR:S49980 | PIR |
| secY | PIR:I40340 | PIR |
| thiX | PIR:I40714 | PIR |
| thrA | PIR:DEFKHG | PIR |
| trpA | PIR:G24723 | PIR |
| trpB | PIR:F24723 | PIR |
| trpC | PIR:E24723 | PIR |
| trpE | PIR:B24723 | PIR |
| trpG | PIR:C24723 | PIR |
| - | YFDA_CORGL | Swissprot |
| - | YPRB_CORGL | Swissprot |
| ackA | ACKA_CORGL | Swissprot |
| amt | AMT_CORGL | Swissprot |
| argB | ARGB_CORGL | Swissprot |
| argD | ARGD_CORGL | Swissprot |
| argJ | ARGJ_CORGL | Swissprot |
| betP | BETP_CORGL | Swissprot |
| brnQ | BRNQ_CORGL | Swissprot |
| clpB | CLPB_CORGL | Swissprot |
| efp | EFP_BRELA | Swissprot |
| ftsZ | FTSZ_BRELA | Swissprot |
| gluA | GLUA_CORGL | Swissprot |
| gluB | GLUB_CORGL | Swissprot |
| gluC | GLUC_CORGL | Swissprot |
| gluD | GLUD_CORGL | Swissprot |
| proB | PROB_CORGL | Swissprot |
| proC | PROC_CORGL | Swissprot |
| thtR | THTR_CORGL | Swissprot |
| trpD | TRPD_CORGL | Swissprot |
| tuf | EFTU_CORGL | Swissprot |
| unkdh | YPRA_CORGL | Swissprot |
| ypt5 | YFZ1_CORGL | Swissprot |
| - | AB009078_1 | Trembl |
| - | CGFDA_2 | Trembl |
| - | CGLYSEG_3 | Trembl |
| accBC | CGU35023_2 | Trembl |
| aecD | CGCSLYS_1 | Trembl |
| amtP | CAJ10319_2 | Trembl |
| amtR | CGL133719_2 | Trembl |
| apt | AF038651_2 | Trembl |
| argC | AF049897_1 | Trembl |
| argF | AF031518_1 | Trembl |
| argG | AF030520_1 | Trembl |
| argH | AF048764_1 | Trembl |
| argR | AF041436_1 | Trembl |
| aroA | AF114233_1 | Trembl |
| aroD | AF036932_1 | Trembl |
| cglIM | CG13922 1 | Trembl |
| cmr | CG43535_1 | Trembl |
| dtsR2 | AB018531_2 | Trembl |
| ectP | CGECTP_1 | Trembl |
| ftsW | BLA242646_2 | Trembl |
| glnD | CAJ10319_4 | Trembl |
| gltB | AB024708_1 | Trembl |
| gltD | AB024708_2 | Trembl |
| hisG | AF050166_1 | Trembl |
| hisH | AF060558_1 | Trembl |
| ilvD | CGL012293_1 | Trembl |
| impA | AF045998_1 | Trembl |
| metA | AF052652_1 | Trembl |
| metB | AF126953_1 | Trembl |
| murC | AB015023_1 | Trembl |
| murG | BLA242646_3 | Trembl |
| ocd | CGL007732_4 | Trembl |
| ppc | A09073_1 | Trembl |
| ppx | CG31224_1 | Trembl |
| pta | CGPTAACKA_1 | Trembl |
| putP | CGPUTP_1 | Trembl |
| rel | AF038651_3 | Trembl |
| sigA | BLSIGAGN_1 | Trembl |
| sigB | BLSIGBGN_2 | Trembl |
| srp | CAJ10319_5 | Trembl |
| ureB | AB029154_3 | Trembl |
| urec | AB029154_4 | Trembl |
| ured | AB029154_8 | Trembl |
| ureE | AB029154_5 | Trembl |
| uref | AB029154_6 | Trembl |
| ureg | AB029154_7 | Trembl |
| ureR | AB029154_1 | Trembl |
| wag31 | BLA242594_1 | Trembl |
| xylB | CGPAN_3 | Trembl |
| yfiH | BLFTSZ_4 | Trembl |
| yhbw | CGCSLYS_3 | Trembl |
| yjcc | CGL133719_1 | Trembl |
| accDA | DE: 19924365.4 | Patent applicatior |
| acp | DE: 10023400.3 | Patent applicatior |
| brnE | DE: 19951708.8 | Patent application |
| brnF | DE: 19951708.8 | Patent application |
| cdsA | DE: 10021828.8 | Patent application |
| cls | DE: 10021826.1 | Patent application |
| cma | DE: 10021832.6 | Patent application |
| dapC | DE: 10014546.9 | Patent application |
| dapF | DE: 199 43 587.1 | Patent application |
| eno | DE: 19947791.4 | Patent application |
| fadD15 | DE: 10021 831.8 | Patent application |
| glk | DE: 19958159.2 | Patent application |
| gpm | DE: 19953160.6 | Patent application |
| lrp | DE: 19947792.2 | Patent application |
| opcA | US: 09/531,267 | Patent application |
| pfk | DE: 19956131.1 | Patent application |
| pfkA | DE: 19956133.8 | Patent application |
| pgi | US: 09/396,478 | Patent application |
| pgsA2 | DE: 10021829.6 | Patent application |
| poxB | DE: 19959327.2 | Patent application |
| ptsH | DE: 10001101.2 | Patent application |
| sdhA | DE: 19959650.6 | Patent application |
| sdhB | DE: 19959650.6 | Patent application |
| sdhC | DE: 19959650.6 | Patent application |
| sod | US: 09/373,731 | Patent application |
| sucC | DE: 19956686.0 | Patent application |
| sucD | DE: 19956686.0 | Patent application |
| tal | US: 60/142,915 | Patent application |
| thrE | DE: 19941478.5 | Patent application |
| zwa1 | DE: 19959328.0 | Patent application |
| zwa2 | DE: 19959327.2 | Patent application |
| zwf | JP: A-092246.61 | Patent application |

In a preferred embodiment of the invention, such arrays can be used for monitoring the transcriptional status of cells on a genomic scale during a fermentation.

In another preferred embodiment of the invention, such arrays can be used for monitoring the transcriptional status of a diagnostic subset of genes during a fermentation.

The arrays according to the invention are preferably used in a method of monitoring a fermentation process by analyzing polynucleotide sequences or fragments thereof of a compound producing microorganism, by the use of an array of DNA probes comprising at least a set that is exactly complementary to select reference sequences of the compound producing microorganism immobilized on a solid support, the different probe DNA's occupying separate cells of the array, which method comprises labeling the reference polynucleotide sequence or fragments thereof, applying the polynucleotide sequence or fragments thereof under hybridization conditions to the array, and observing the location and the intensity of the label on the surfaces associated with particular members of the probe DNA's.

In a preferred embodiment the polynucleotide sequence of Corynebacterium glutamicum strain separated from a fermentation broth is analyzed.

In another embodiment the polynucleotide sequence of an Escherichia coli strain separated from a fermentation broth is analyzed.

The array is used to monitore the process related target genes of compound producing microorganisms in the fermentation process.

In a preferred embodiment the fermentation process of compound production is monitored by said method including the following steps:
fermentation of the bacteria producing L-amino acid(s), vitamins, metabolites, antioxidants, cellular or secreted proteins, pigments, nucleotides, sugars or peptides
isolation of the microorganism cells during the fermentation and preparation of the cellular ribonucleic acid (RNA)
labeling of the isolated RNA with a known technique like a direct labeling method or an incorporation of labeled nucleotides during by generation of a copy of the isolated RNA, e.g. to cDNA/cRNA.
subsequent hybridisation of the labeled RNA/cDNA/cRNA to an array of single or double stranded nucleic acid probes for the detection of transcripts of coryneform or coliform bacteria
detection of the hybridization pattern of the signals by known methods
comparison of obtained hybridization patterns
usage of the obtained results for improving processes and productivity

### Examples

### Example 1

Manufacture of Arrays:

The primers for the PCR amplification of the probe DNA is chosen using the Primer3 software with the default settings. The only exemption is the product size, which settings are set to 200 - 3000 base pairs with an optimum product size of 500 base pairs (Steve Rozen, Helen J. Skaletsky (1998) Primer3. Code available at http://www-genome.wi.mit.edu/genome_software/other/primer3.html.) On account of the sequences of the probe genes known from databases, as an example the following oligonucleotides are selected for the polymerase chain reaction of the aceA gene:

aceA1:

5` ccacacctaccctgaccagt 3`

aceA2:

5` ggctcgagaccattcttgac 3`

The chosen primers are synthesized by MWG Biotech (Ebersberg, Germany) and the PCR reactions for all genes is carried out according to the standard PCR method of Innis et al. (PCR protocols. A guide to methods and applications, 1990, Academic Press) using Taq polymerase from Boehringer Mannheim (Germany, Product Description Taq DNA Polymerase, Product No. 1 146 165). Amongst other sources, one skilled in the art will find further instructions for the amplification of DNA sequences with the aid of polymerase chain reaction (PCR) in the Handbooks by Gait: Oligonucleotides synthesis: a practical approach (IRL Press, Oxford, UK, 1984) and by Newton and Graham: PCR (Spektrum Akademischer Verlag, Heidelberg, Germany, 1994). Chromosomal DNA as template for the PCR reaction is isolated from the strain ATCC 13032 by the method of Eikmanns et al. (Microbiology 140: 1817-1828 (1994)). With the aid of the polymerase chain reaction the primers permit the amplification of internal fragment of the selected genes that can be used as a hybridization probe which is immobilized on a microarray. The thus amplified products are tested electrophoretically in a 1.0% agarose gel.

The PCR products are desalted and purified using Multiscreen PCR plates (Cat. No. MANU 030 10, Millipore Corporation, Bedford, MA, USA) according to the manufacturers instructions. These probe DNA's are mixed with spotting buffer and printed onto ArrayLink hydrophob microarray substrates (GeneScan Europe AG, Freiburg, Germany) using a Microgrid Microarray Spotter (Biorobotics, Cambridge, UK). The microarrays are produced following the manufacturers instructions.

### Example 2

### L-amino acids fermentation

For production of L-lysine the C. glutamicum strains ATCC13032, DSM5715 and ATCC21513 are cultivated in a nutrient medium suitable for the production of L-lysine and the L-lysine content in the culture supernatant is determined. The strains ATCC13032 and ATCC21513 can be obtained from the American Type Culture Collection (Manassas, VA, USA), the strain DSM5715 is described in EP-B-0435132.

For the purpose of L-Lysine production the strain is first of all incubated for 24 hours at 33°C on an agar plate (brain-heart agar, starting from this agar plate culture a preculture is inoculated (10 ml of medium in a 100 ml Erlenmeyer flask). The full medium CgIII is used as medium for the preculture.

| medium Cg III | |
|---|---|
| NaCl | 2.5 g/l |
| Bacto-Peptone | 10 g/l |
| Bacto-Yeast Extract | 10 g/l |
| Glucose (autoclaved separately) | 2% (w/v) |
| The pH value is adjusted to pH 7.4 | The preculture is incubated for 16 hours at 33°C at 240 rpm on a shaker table. From this preculture a main culture is inoculated so that the initial OD (660 nm) of the main culture is 0.1 OD. The medium MM is used for the main culture. |

| Medium MM | |
|---|---|
| CSL (Corn Steep Liquor) | 5 g/l |
| MOPS | 20 g/l |
| Glucose (autoclaved separately) | 50 g/l |
| Salts: (NH4)2SO4) | 25 g/l |
| KH2PO4 | 0.1 g/l |
| MgSO4.7H2O | 1.0 g/l |
| CaCl2.2H2O | 10 mg/l |
| FeSO4.7H2O | 10 mg/l |
| MnSO4.H2O | 5.0 mg/l |
| Biotin (sterile filtered) | 0.3 mg/l |
| Thiamine.HCl (sterile filtered) | 0.2 mg/l |
| Homoserine (sterile filtered) | 0.1 g/l |
| Leucine (sterile filtered) | 0.1 g/l |
| CaCO3 | 25 g/l |
| | CSL, MOPS and the salt solution are adjusted with ammonia water to pH 7 and autoclaved. The sterile substrate solutions and vitamin solutions as well as the dry autoclaved CaCO₃ are then added. |

Cultivation is carried out in a 10 ml volume in a 100 ml Erlenmeyer flask equipped with baffles. The cultivation is carried out at 33°C and 80% atmospheric humidity.

After 48 hours the OD is determined at a measurement wavelength of 660 nm with a Biomek 1000 (Beckmann Instruments GmbH, Munich). The amount of L-lysine formed is determined by ion exchange chromatography and post-column derivatisation with ninhydrin detection using an amino acid analyzer from Eppendorf-BioTronik (Hamburg, Germany).

The results of the experiment are shown in Table 1.

**Table 1**

| Strain | OD(660) | L-lysine-HCl g/l |
|---|---|---|
| ATCC13032 | 12.8 | 0.1 |
| DSM5715 | 8.2 | 12.8 |
| ATCC21513 | 8.4 | 13.4 |

### Example 3:

### Isolation and labeling of RNA from C.glutamicum:

From the *C.glutamicum* cultures described in Example 2, total RNA is isolated after 12, 24, 36 and 48 hours. Therefore an appropriate volume, e.g.5 ml of such a culture is mixed with the some volume of ice cold 20mM NaN₃ (Catalog number 1.06688.0100, Merck, Darmstadt, Germany). The cells are harvested by centrifugation for 10 minutes at 10000 x g. The RNA extraction is done using a Ribolyser machine (Catalog number HB6000-120, Hybaid, Heidelberg, Germany) an the Hybaid RiboLyser™ Blue Kit (Catalog number RY61100 Hybaid, Heidelberg, Germany). This crude RNA-preparation is further purified with the SNAP total RNA isolation kit from Invitrogen Corporation (Carlsbad, CA, USA; Cat. No. K1950-05). By this treatment DNA contaminations in the RNA preparation are removed by digestion with DNAseI followed by RNA purification on silica membrane spin columns, done according to the manufacturers instructions. 50-100µg of this RNA preparation is used for one labeling procedure.

Total bacterial RNA is labeled by generation of a single stranded copy DNA (cDNA). For the labeling 100 µg total RNA are mixed with 10µg of oligonucleotide primers as starting point for the reverse transcription. These primers consist of an equimolar mixture of random hexamers and random octamers. The random primers are synthesized by MWG (Ebersberg, Germany). The incorporation of the fluorescent dyes and the purification of the labeled cDNA is done using the Atlas™ Glass Fluorescent Labeling Kit (Cat. No. K1037-1, Clontech, Heidelberg, Germany) following the manufacturers instructions.

Using the described protocol, the cDNA of the no L-lysine producing strain ATCC13032 is labeled with the fluorescent dye Cy3. The cDNA of the L-lysine producing strain ATCC21513 is labeled with the fluorescent dye Cy5.

### Example 4

### Comparative analysis of the transcriptional status of the strains ATCC13032 and ATCC21513

As described in Example 3, for each strain the total cellular RNA is isolated and labeled at different time points during the fermentation. For the analysis of differences in the transcriptional status, the labeled cDNA of both strains is hybridized competitively for each time point on the arrays described in example 1. For the experienced user beneath other sources, the principles and further technical and methodological details are described in the book of M. Schena, (DNA Microarrays, Editor: M. Schena, Oxford Universtity Press, 1999)

The hybridization is done using the Atlas™ Glass Hybridization Chamber and GlassHyb Solution (Catalog numbers 7899-1 and 8016-1 Clontech, Heidelberg, Germany). The slides are scanned using a Scanarray 4000 confocal microarray scanner (GSI-Lumonics, Billerica, MA, USA) following the manufacturers instructions. The aquired images are further analyzed using the QuantArray Software, provided together with the scanner.

The fluorescence intensity for each spot and each fluorescence dye is calculated separately. The results of each data point of the single experiments are plotted against each other. Signals that give a data point that is more than a factor of 1.5 away from the correlation line are regulated differentially in the two strains.

Examples of genes that are upregulated or downregulated at one or more time points and the maximum fold change difference in the expression level in the L-lysine producing strain ATCC21513 compared to the no L-lysine producing strain ATCC13032 are listed in Table 2:

**Table 2**

| **Genes higher expressed in ATCC21513 compared to ATCC13032** | | **Genes lower expressed in ATCC21513 compared to ATCC13032** | |
|---|---|---|---|
| gap | 3 fold | pgi | 4 fold |
| lysC | 4 fold | fda | 2 fold |
| dapA | 4 fold | pyk | 5 fold |
| lysE | 2 fold | glt | 3 fold |
| sucC | 4 fold | icd | 2 fold |
| dapC | 3 fold | rel | 2 fold |
| ptsM | 5 fold | ilvC | 2 fold |

### Example 5

### Monitoring a fermentation by comparison of gene expression patterns

The gene expression patterns described in the Examples 2-4 can be used to monitore a fermentation process.

Therefore the RNA of cells from a good fermentation, i.e. with the expected L-lysine productivity, is prepared and labeled as described in Example 3. The hybridization pattern of the labeled cDNA resulting from one or more combined RNA preparations from a good fermentation is compared with the hybridization pattern of a cDNA resulting from an other fermentation that is to be monitored. The hybridization patterns are basically obtained as described in Example 4. In order to achieve shorter analysis times , the amount of cDNA can be increased and the hybridization time can be decreased.

The sample that is monitored is taken at about the same time point and the same optical density as the sample from the good reference fermentation that is used as reference sample.

The expression data are analyzed by a scatter plot analysis. Signals that give a data point that is more than a factor of about 1.5 - 2.0 away from the correlation line are regulated differentially in the two fermentations. Such differences in gene expression indicate a problem with the fermentation efficiency in respect to product formation or biomass formation.

If more than > 0 - 6 %, preferable > 0 - 3% of the genes are located more than the factor of 2 away from the correlation line, the fermentation is good.

If more than 3 - 15 %, preferable 3 - 8 % of the genes are located more than the factor of 2 away from the correlation line, the fermentation might give low product, biomass or sugar conversion yields.

If more than 15% of the genes are located more than the factor of 2 away from the correlation line, the fermentation will give low product yields.

Within these gene expression patterns that can be correlated to the fermentation yield, there are also single genes that can be used to monitor a fermentation. Changes in the individual gene expression level of these genes indicate a problem in the fermentation process. An example is the glt gene, whose expression is about 3-fold decreased in a L-Lysin producing strain compared to a wild type as shown in example 4. If this ration is increased to more than 5-fold weaker expression, the L-Lysine yield obtained as described in Example2 will decrease for about 5% from 13.4 g/l to 13.1 g/l. Probes for such genes can be immobilized on diagnostic DNA-arrays and be used for monitoring a fermentation process.

### Example 6

### Improving a Fermentation by inactivation of the pgi gene

The genes described in Example4 are differentially regulated in a L-lysine producing C.glutamicum strain. In order to show the positive effect of this differential regulation on L-lysine production, as an example the pgi gene is inactivated in the L-lysine producing strain DSM5715.

Therefore an integration vector for the integration mutagenesis of the pgi gene is constructed.

Chromosomal DNA is isolated from the strain ATCC 13032 by the method of Eikmanns et al. (Microbiology 140: 1817 - 1828 (1994)). On account of the sequence of the pgi gene for C. glutamicum, the following oligonucleotides are selected for the polymerase chain reaction:

pgi-int1:

5` GACCTCGTTTCTGTGTTGG 3`

pgi-int2:

5` TGACTTGCCATTTGATTCC 3`

The represented primers are synthesized by MWG Biotech (Ebersberg, Germany) and the PCR reaction is carried out according to the standard PCR method of Innis et al. (PCR protocols. A guide to methods and applications, 1990, Academic Press) using Taq polymerase from Boehringer Mannheim (Germany, Product Description Taq DNA Polymerase, Product No. 1 146 165). With the aid of the polymerase chain reaction the primers permit the amplification of a 516 bp large internal fragment of the pgi gene. The thus amplified product is tested electrophoretically in a 0.8% agarose gel.

The amplified DNA fragment is ligated into the vector pCR2.1-TOPO (Mead at al. (1991) Bio/Technology 9:657-663) using the TOPO TA Cloning Kit from Invitrogen Corporation (Carlsbad, CA, USA; Cat. No. K4500-01).

The E. coli strain TOP10 is then electroporated with the ligation batch (Hanahan, In: DNA cloning. A practical approach. Vol. I. IRL-Press, Oxford, Washington DC, USA, 1985). Plasmid-carrying cells are selected by plating out the transformation batch onto LB agar (Sambrook et al., Molecular cloning: a laboratory manual. 2nd Ed. Cold Spring Harbor Laboratory Press, Cold Spring Harbor, N.Y., 1989) that has been supplemented with 50 mg/l of kanamycin. Plasmid DNA is isolated from a transformant using the QIAprep Spin Miniprep Kit from Qiagen and is checked by restriction with the restriction enzyme EcoRI followed by agarose gel electrophoresis (0.8%). The plasmid is named pCR2.1pgiint.

The vector pCR2.1pgiint is electroporated into Corynebacterium glutamicum DSM 5715 according to the electroporation method of Tauch et. al. (FEMS Microbiological Letters, 123:343-347 (1994)). The strain DSM 5715 is an AEC-resistant L-lysine producer. The vector pCR2.1pgiint cannot replicate independently in DSM5715 and thus only remains in the cell if it has integrated into the chromosome of DSM 5715. The selection of clones with pCR2.1pgiint integrated into the chromosome is made by plating out the electroporation batch onto LB agar (Sambrook et al., Molecular cloning: a laboratory manual. 2nd Ed. Cold Spring Harbor Laboratory Press, Cold Spring Harbor, N.Y.) that has been supplemented with 15 mg/l of kanamycin.

In order to demonstrate the integration the pgiint fragment is labeled using the Dig Hybridisation Kit from Boehringer according to the method described in "The DIG System User's Guide for Filter Hybridization" published by Boehringer Mannheim GmbH (Mannheim, Germany, 1993). Chromosomal DNA of a potential integrant is isolated according to the method of Eikmanns et al. (Microbiology 140: 1817 - 1828 (1994)) and is in each case cleaved with the restriction enzymes SacI, EcoRI and HindIII. The resultant fragments are separated by means of agarose gel electrophoresis and hybridized at 68°C using the Dig Hybridisation Kit from Boehringer. The plasmid pCR2.1pgiint has inserted itself into the chromosome of DSM5715 within the chromosomal pgi gene. The strain is designated DSM5715::pCR2.1pgiint.

The C. glutamicum strain DSM5715::pCR2.1pgiint is cultivated in a nutrient medium suitable for the production of L-lysine and the L-lysine content in the culture supernatant is determined.

For this purpose the strain is first of all incubated for 24 hours at 33°C on an agar plate with the corresponding antibiotic (brain-heart agar with kanamycin (25 mg/l). Starting from this agar plate culture a preculture is inoculated (10 ml of medium in a 100 ml Erlenmeyer flask). The rich medium CgIII described in Example 2 is used as medium for the preculture.

Cultivation is carried out in MM-Medium described in Example 2 with 10 ml volume in a 100 ml Erlenmeyer flask equipped with baffles. Kanamycin is added (25 mg/l). The cultivation is carried out at 33°C and 80% atmospheric humidity.

After 48 hours the OD is determined at a measurement wavelength of 660 nm with a Biomek 1000 (Beckmann Instruments GmbH, Munich). The amount of L-lysine formed is determined by ion exchange chromatography and post-column derivatisation with ninhydrin detection using an amino acid analyzer from Eppendorf-BioTronik (Hamburg, Germany).

The results of the experiment are shown in Table 3.

**Table 3**

| Strain | OD(660) | L-lysine-HCl g/l |
|---|---|---|
| DSM5715 | 8.2 | 13.7 |
| DSM5715::pCR2.1pgiint | 7.9 | 18.8 |

### Example 7

### Improving a Fermentation by overexpression of the gap gene

The genes described in Example4 are differentially regulated in a L-lysine producing C.glutamicum strain. In order to show the positive effect of this differential regulation on L-lysine production, as an example the gap gene is overexpressed in the L-lysine producing strain DSM5715.

Therefore the gap gene is cloned in the vector pJCl. Chromosomal DNA from *Corynebacterium glutamicum* ATCC 13032 is isolated as described in example 5. A DNA fragment bearing the gap gene is amplified by polymerase chain reaction. The following primers are used for this purpose:

gapA1

5`-TGCTCTAGATTGAAGCCAGTGTGAGTTGC-3`

gapA2

5`-TGCTCTAGAGATGACACATCACCGTGAGC-3`

The primers illustrated are synthesized by MWG Biotech (Ebersberg, Germany) and the PCR reaction is carried out by the standard PCR method of Innis et al.(PCR protocol. A guide to methods and applications, 1990, Academic Press). The primers enabled amplification to be effected of a DNA fragment with a size of about 1520 bp and bearing the gap gene of *Corynebacterium glutamicum.*

After separation by gel electrophoresis, the PCR fragment is isolated from the agarose gel using a QiaExII Gel Extraction Kit (Product No. 20021, Qiagen, Hilden, Germany).

The *E. coli - C. glutamicum* shuttle vector pJC1 (Cremer et al., 1990, Molecular and General Genetics 220: 478 - 480) is used as a vector. This plasmid is completely cleaved with the restriction enzyme BamHI, is treated with Klenow polymerase (Roche Diagnostics GmbH, Mannheim, Germany) and is subsequently dephosphorylated with shrimp alkaline phosphatase (Roche Diagnostics GmbH, Mannheim, Germany, product description SAP, Product No. 1758250).

The gap fragment obtained in this manner is mixed with the prepared vector pJC1 and is ligated with the aid of a SureClone Ligation Kit (Amersham Pharmacia Biotech, Uppsala, Sweden) according to the manufacturer's instructions. The ligation batch is transformed in the *E. coli* strain DH5 (Hanahan, in: DNA cloning. A practical approach. Vol. I. IRL Press, Oxford, Washington DC, USA). Plasmid-bearing cells are selected by plating out the transformation batch on LB agar (Lennox, 1955, Virology, 1:190) with 50 mg/l kanamycin. After incubation overnight at 37°C, recombinant individual clones are selected. Plasmid DNA is isolated from a transformant using a Qiaprep Spin Miniprep Kit (Product No. 27106, Qiagen, Hilden, Germany) according to the manufacturer's instructions and is cleaved with the restriction enzyme XbaI in order to investigate the plasmid by subsequent agarose gel electrophoresis. The plasmid obtained is designated as pJClgap.

The *C. glutamicum* strains ATCC13032 and DSM5715 are transformed with the plasmid pJC1gap using the electrophoration method described by Liebl et al. (FEMS Microbiology Letters, 53:299-303 (1989)). The transformants are selected on LBHIS agar consisting of 18.5 g/l brain-heart infusion bouillon, 0.5 M sorbitol, 5 g/l bacteriological trypton, 2.5 g/l bacteriological yeast extract, 5 g/l NaCl and 18 g/l bacteriological agar which is supplemented with 25 mg/l kanamycin. Incubation is effected for 2 days at 33°C.

Plasmid DNA is isolated from each transformant by the usual methods (Peters-Wendisch et al., 1998, Microbiology, 144, 915 - 927), is cut with the restriction endonuclease XbaI and the plasmid is investigated by subsequent agarose gel electrophoresis. The strain obtained is designated as DSM5715/pJC1gap.

The *C. glutamicum* strains DSM5715 and DSM5715/pJC1gap are cultivated as described in Example 2.

After 48 hours, the OD and the L-lysine content in the culture supernatant is determined as described in Example 2

The results of the experiment are given in Table 4.

**Table 4**

| Strain | OD(660 nm) | L-lysine-HCl (g/l) |
|---|---|---|
| DSM5715 | 8.1 | 13.6 |
| DSM5715//pJC1gap | 7.6 | 14.4 |

## Claims

1. An array of DNA probes immobilized on a solid support, said array having at least 10 probes and no more than 200.000 different DNA probes 15 to 4.000 nucleotides in length occupying separate known sites in said array, said DNA probes comprising at least a set that is exactly complementary to selected reference sequences of a compound producing microorganism

2. The array of claim 1, wherein said reference sequence is a single-stranded nucleic acid and probes complementary to the single-stranded nucleic acid or to a cDNA or cRNA of the single-stranded nucleic acid of said reference are in said array.

3. The array of claim 1, wherein the reference sequence is from Corynebacterium glutamicum.

4. The array of claim 1, wherein the reference sequence is from Escherichia coli.

5. A method of monitoring a fermentation process by analyzing parts of a polynucleotide sequence of a compound producing microorganism, by the use of an array of DNA probes comprising a least a set that is complementary to selected reference sequences of the compound producing microorganism immobilized on a solid support, the different probe DNA's occupying separate cells of the array, which method comprises labeling the polynucleotide sequence or fragments thereof, applying the polynucleotide sequence or fragments thereof under hybridization conditions to the array, and observing the location and the intensity of the label on the surface associated with particular members of the probe DNA's.

6. A method of claim 5, wherein a polynucleotide sequence of a Corynebacterium glutamicum strain separated from a fermentation broth is analyzed.

7. A method of claim 5, wherein a polynucleotide sequence of an Escherichia coli strain separated from a fermentation broth is analyzed.

8. A method of claim 5, wherein the array is used to monitore process related target genes of compound producing microorganisms in the fermentation process.

9. A method of claim 8, wherein a L-lysine producing Corynebacterium is used.

10. A method of monitoring a fermentation process of compound producing microorganisms, **characterized in that** the following steps are performed:
a) fermentation of the microorganisms,
b) isolation of the microorganism cells during the fermentation and preparation of the cellular ribonucleic acid (RNA)
c) labeling of the isolated RNA with a known technique like a direct labeling method or a reverse transcription of the isolated RNA to cDNA/cRNA with a concomitant incorporation of labeled nucleotides
d) subsequent hybridisation of the labeled RNA/cDNA/cRNA to an array of single or double stranded nucleic acid probes for the detection of transcripts of coryneform or coliform bacteria
e) detection of the hybridization pattern of the signals by known methods
f) comparison of obtained hybridization patterns
g) usage of the obtained results for improving a fermentation process
